# EUROPÄISCHE PATENTSCHRIFT

(11) **EP 0 476 669 B1**
(45) Veröffentlichungstag und Bekanntmachung des Hinweises auf die Patenterteilung: **03.05.1995**
(21) Anmeldenummer: 91115973.9
(22) Anmeldetag: 19.09.1991
(51) Int. Cl.: C12Q 1/44, C12Q 1/61

(54) **Verfahren zur Bestimmung von Phospholipase A in Körperflüssigkeiten**
Method for determination of phospholipase A in body fluids
Méthode pour détermination de phospholipase A dans les fluides corporels

(30) Priorität: 20.09.1990 DE 4029845
(43) Veröffentlichungstag der Anmeldung: 25.03.1992
(73) Patentinhaber: BOEHRINGER MANNHEIM GMBH, 68298 Mannheim (DE)
(72) Erfinder: Neumann, Ulrich, Dr., W-8120 Weilheim (DE); Schmidt, Gudrun, W-8139 Bernried (DE); Junius-Comer, Martina, Dr., W-8127 Iffeldorf (DE)
(74) Vertreter: Huber, Bernhard, Dipl.-Chem.

(56) Entgegenhaltungen:
- EP-A- 0 331 167
- EP-A- 0 337 005
- EP-A- 0 399 379

## Beschreibung

Die Erfindung betrifft ein Verfahren zur Bestimmung von Phospholipase A in Körperflüssigkeiten, insbesondere Serum.

In der Natur kommen Phospholipasen ubiquitär in den verschiedensten Organismen vor. Besonders bekannt sind sie aus Bienengift und den verschiedenen Schlangengiften.

Die Bestimmung der Phospholipase A (PLA) kann nach der Literatur zur Diagnostik von verschiedenen lebensbedrohenden Erkrankungen dienen. Die Einteilung der Phospholipasen erfolgt nach deren Einwirkungsstelle am Lecithin-Molekül. Die Phospholipase A spaltet die Esterbindung mit dem Fettsäuremolekül, die Phospholipase A₁ die α-ständige, die Phospholipase A₂ die β-ständige.

Im menschlichen Pankreas wird PLA in erheblichen Mengen gebildet und ins Duodenum sezerniert, wo täglich ca. 4 g Nahrungslecithin und zusätzlich ca. 10 g körpereigenes Lecithin gespalten werden. Die pancreatische PLA ist mit etwa 130 Aminosäuren ein ausgesprochen kleines Enzym.

Schon früh wurde nach einer klinischen Relevanz der PLA gesucht, wie sie die anderen Pancreasenzyme Lipase und Amylase haben. Wegen der Gefährlichkeit verschiedener PLA-Enzyme (durch Einwirkung der PLA₂ auf Lecithine entstehen unter Abspaltung einer Fettsäure in 2-Stellung die hochtoxischen Lysolecithine) wurde insbesondere an die Möglichkeit einer Differenzierung des Schweregrades von Pancreatitis geglaubt. Die akute Pancreatitis kann nämlich als relativ harmlose oedematöse oder höchst gefährliche nekrotisierende Form vorliegen, deren Differentialdiagnose klinisch sehr wichtig ist. Von Hoffmann et al. (J.Clin.Chem.Clin.Biochim. 23 (1985), Seite 582) konnte anhand von etwa 50 Seren mit klinisch gesicherter Diagnose gezeigt werden, daß tatsächlich bei der akuten haemorhargischen Pancreatitis über die PLA-Enzymaktivität eine Prognose über den Schwereverlauf der Krankheit gemacht werden kann. Durch weiteres Untersuchungsmaterial wurde im Laufe der Zeit deutlich, daß auch andere Erkrankungen mit PLA-Erhöhungen einhergehen, wobei auch hier der PLA-Spiegel immer ein Indikator für die Schwere der Krankheit ist. Beispielhaft seien hier genannt Lungenversagen, ARDS (MacGuire et al., J.Clin.Invest. 69 (1982), 543-553), Sepsis (Vadas P., J.Lab.Clin.,Med. 104 (1984), 873), allergischer Schock, progedient chronische Polyarthritis (Pruzanski et al., J.Rheumatol. 12 (1985), 211) sowie Asthma bronchiale.

Bei diesen Krankheitsbildern lag es nahe, daß die Ursache nicht mit einer pancreatischen PLA in Verbindung gebracht werden konnte. Es ist beschrieben, daß aus der Synovialflüssigkeit eines Patienten mit progredient chronischer Polyarthritis eine PLA isoliert worden ist, die nicht mit einem Antiserum gegen die humane Pancreas-PLA immunologisch reagierte. Hoffmann et al. konnte zeigen, daß die PLA in Humanseren ein anderes pH-Optimum als die humane Pancreas-PLA hat (Dt.Ges.f.Klin.Chemie e.V. - Mitteilungen 5/87 (1987), 226).

Der erste Test auf PLA in Humanseren wurde von Zieve und Vogel 1961 beschrieben (J.Lab.Clin.Invest. 57 (1961), 586). Es war ein titrimetrischer Test, der pro Bestimmung eine Inkubationszeit von 18 Stunden bei einer Temperatur von 55°C benötigte. Es wurden in der Folgezeit weitere PLA-Bestimmungsmethoden publiziert, wie Messung über radioaktiv markierte Substrate, Extraktion und Photometrie der gebildeten freien Fettsäuren, Fluorimetrie u.a.. Diese Verfahren waren jedoch allesamt äußerst zeitaufwendig und kompliziert, so daß hier eine routinemäßige Durchführung nicht möglich war.

Es war daher Aufgabe der vorliegenden Erfindung, einen PLA-Test bereitzustellen, der die analytische und apparative Problematik der bekannten PLA-Tests vermied und mit einem üblichen Laborphotometer durchgeführt werden kann.

Gelöst wird diese Aufgabe durch ein Verfahren zur Bestimmung von Phospholipase A (PLA) in Körperflüssigkeiten, insbesondere Serum, bei dem man die Körperflüssigkeit mit einer Mischung, welche ein Fettsäurereste aufweisendes Substrat für die Phospholipase und mindestens zwei Emulgatoren verschiedener chemischer Klasse, nämlich mindestens einen neutralen Emulgator und ein Gallensäuresalz, enthält, inkubiert und die Menge der durch Substratspaltung gebildeten freien Fettsäuren nach an sich bekannten Methoden bestimmt. Der neutrale Emulgator kann im erfindungsgemäßen Verfahren hydrophil oder auch lipophil sein.

Als Substrat kann natürliches Lecithin eingesetzt werden, wobei sich jedoch nicht alle Lecithine gleich gut eignen. Ein hoher Anteil an ungesättigten Fettsäuren im Lecithinmolekül scheint eine Voraussetzung für gute Testergebnisse zu sein. In einer bevorzugten Ausführungsform der Erfindung setzt man als natürliches Lecithinsubstrat Lecithin aus Sojabohnen oder Lipoid S20 ein.

Abgesehen von natürlichem Lecithin kann auch ein künstliches Substrat eingesetzt werden, welches der allgemeinen Formel 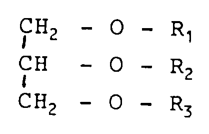
entspricht, wobei R₁ und R₂ einen Alkylrest mit bis zu 20 C-Atomen oder eine Carbonylgruppe mit bis zu 20 C-Atomen bedeuten, unter der Voraussetzung, daß mindestens einer der beiden Reste eine Carbonylgruppe ist, und R₃ -SO₃H bedeutet.

In einer bevorzugten Ausführungsform der Erfindung verwendet man als ersten Emulgator ein Alkylglucosid, Polyether, DMSO oder Triton®X-100 (Alkylphenylpolyethylenglycol). Als zweiter Emulgator wird ein Gallensäuresalz, vorzugsweise Taurodesoxycholat oder Desoxycholat verwendet. Besonders bevorzugt werden die Kombinationen von Taurodesoxycholat mit Octyl-β-D-glucopyranosid, Polyoxyethylenether, DMSO oder Triton®X-100 verwendet.

Im erfindungsgemäßen Verfahren ist es bevorzugt, ein lyophilisiertes Substrat zu verwenden und dieses in Puffer, insbesondere in HEPES-Puffer zu lösen. Hierdurch wird es erleichtert, standardisierte Mengen an Substrat einzusetzen, wobei die Mengenabmessung besonders einfach ist.

Die Bestimmung der gebildeten freien Fettsäuren kann prinzipiell auf verschiedene Arten erfolgen, nämlich im Prinzip durch Umsestzung der freien Fettsäure in Anwesenheit von Coenzym A und ATP durch Acyl-CoA-Synthetase zu Acyl-CoA, AMP und Pyrophosphat und Nachweis einer der gebildeten Komponenten. Hierbei ist sowohl die Bestimmung über Acyl-CoA denkbar, bei dem Acyl-CoA durch Acyl-CoA-Oxidase in Gegenwart von Sauerstoff zu 2,3-Transenoyl-CoA und H₂O₂ umgesetzt wird und das gebildete H₂O₂ über eine Farbstoffbildungsreaktion in Anwesenheit von Peroxidase nachgewiesen wird. Eine weitere Möglichkeit liegt in der Bestimmung über das gebildete Pyrophosphat (PPi). Hierbei wird PPi durch eine Reihe von enzymatisch katalysierten Umsetzungen zu Gluconat-6-Phosphat umgesetzt. Eine weitere Möglichkeit, die für das erfindungsgemäße Verfahren bevorzugt ist, liegt in der Bestimmung über das gebildete AMP. AMP wird hierbei in Anwesenheit von ATP durch Myokinase zu ADP umgesetzt, das wiederum in Anwesenheit von Phosphoenolpyruvat durch Pyruvatkinase zu ATP und Pyruvat umgesetzt wird, wonach das gebildete Pyruvat in Anwesenheit von NADH durch Lactatdehydrogenase in Lactat und NAD umgewandelt wird. Die Bestimmung erfolgt durch Messung der Extinktionsänderung nach Zusatz der PLA-haltigen Körperflüssigkeit zu der Substrat und Emulgatoren enthaltenden Mischung, welche vorzugsweise auch gleich die Reagenzien für die weiteren Umsetzungen von AMP zu Lactat enthält. Gegenüber einer Eichkurve, welche mit einer bekannten Menge PLA aufgestellt wurde, kann dann die Konzentration in der Körperflüssigkeit bestimmt werden.

In wiederum einer weiteren bevorzugten Ausführungsform der Erfindung wird die Bestimmung der gebildeten Fettsäuren in Gegenwart von TRA-Puffer durchgeführt.

Um eine weitere Verfahrenserleichterung zu bewirken, wird vorzugsweise bei der Testdurchführung ein aufhellendes System eingesetzt, das die Farbbildung jedoch nicht beeinträchtigt. In einer besonderes bevorzugten Ausführungsform setzt man hierzu Triton®X-100 zu, falls dieses nicht sogar bereits als Emulgator eingesetzt wurde.

Mit der erfindungsgemäßen Verfahrensführung wird es ermöglicht, auf leichte Weise und routinemäßig die Menge von PLA in Probenflüssigkeit zu bestimmen. Insbesondere ist diese Methode auch leicht auf einem Analysenautomaten durchzuführen, was den Einsatz des Verfahrens im klinischen Labor sehr erleichtert.

Die folgenden Beispiele sollen die Erfindung weiter erläutern.

### Abkürzungen:

- TRA: : Triethanolamin-hydrochlorid
- DMSO: : Dimethylsulfoxid
- TDCh: : Taurodesoxycholat
- PEP: : Phosphoenolpyruvat
- PK: : Pyruvatkinase
- LDH: : Lactatdehydrogenase
- ACS: : Acylcoenzym A-Synthetase
- NaDCh: : Natriumdesoxycholat

### Beispiel 1

### Herstellung der Emulsion

### Emulgatoren

### Beispiel 1.1: Octylglucosid

35 mg Lecithin aus Sojabohnen werden mit 4,25 ml HEPES-Puffer (pH = 7,9, 100 mmol/l), 0,250 ml TDCh-Lösung (120 mmol/l), 0,250 ml einer Lösung von Ca²⁺- und Mg²⁺-Ionen (200 bzw. 400 mmol/l) sowie 0,250 ml Octylglucosidlösung (100 mmol/l) ca. 2 h gerührt. Anschließend wird die Emulsion 2 + 1 mit Octylglucosidlösung verdünnt.
Die Extinktion der Emulsion wird bei 365 nm gegen Luft gemessen. Sie beträgt 1,456.

### Testansatz:

Die Bestimmungen werden bei 37°C durchgeführt. Gemessen wird bei einer Wellenlänge von 365 nm gegen Luft.
Zu 750 »l der oben genannten Lösung werden 25 »l einer CoA-Lösung (90 mmol/l), 75 »l einer Coenzymlösung (ATP: 145 mmol/l, PEP: 55 mmol/l, NADH 9,4 mmol/l) sowie 50 »l einer Enzymlösung (Myokinase: 165 kU/l, LDH: 377 kU/l, PK: 141 kU/l, ACS: 5,6 kU/l) gegeben. Als Probe werden 50 »l eines PLA-haltigen Humanserums eingesetzt.

### Beispiel 1.2: Brij®35 (Polyethylenglycoldodecylether)

35 mg Lecithin werden mit 0,250 ml Taurodesoxycholat-Lösung und 4,5 ml HEPES-Puffer (pH = 7,9, 100 mmol/l) versetzt und unter leichtem Erwärmen gerührt.
Nach 30 min werden 0,250 ml einer Lösung von Ca²⁺- und Mg²⁺-Ionen (200 mmol/l bzw. 400 mmol/l) sowie 0,250 ml Brij®35-Lösung (200 g/l) zugesetzt.
Die Emulsion wird anschließend mit HEPES-Puffer verdünnt (2 Teile Emulsion, 1 Teil Puffer).

Die Extinktion der Emulsion beträgt bei 365 nm gegen Luft 2,243.
Der Testansatz wird wie in Beispiel 1.1 angegeben durchgeführt.

### Beispiel 1.3: Dimethylsulfoxid (DMSO)

35 mg Lecithin werden mit 4,5 ml HEPES-Puffer (pH 7,9, 100 mmol/l) und 0,250 ml Taurodesoxycholat-Lösung gerührt und anschließend mit 0,250 ml einer Lösung von Ca²⁺- und Mg²⁺-Ionen (200 mmol/l, bzw. 400 mmol/l) versetzt. Anschließend wird die Emulsion mit 2 »l DMSO-Lösung (Konz. in der Emulsion: 5 mmol/l) versetzt. Die Extinktion der Emulsion beträgt bei 365 nm gegen Luft 2,573.
2 ml Emulsion werden mit 0,100 ml einer Lösung von 200 mmol/l CaCl₂·2 H₂O, 400 mmol/l MgCl₂·6 H₂O und 200 mmol/l Triton® X-100 versetzt.
Die Extinktion der Emulsion beträgt bei 365 nm gegen Luft 1,320.
Der Testansatz wird wie in Beispiel 1.1 angegeben durchgeführt.

### Beispiel 2

### PLA-Substrate

### Beispiel 2.1:

### PLA-Substrat:

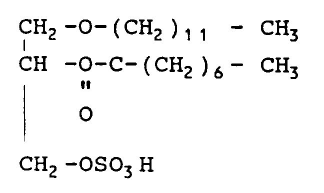

Herstellung der Emulsion: Das PLA-Substrat wird mit 1,5 ml einer Lösung von 125 mmol/l TRIS/HCl, 4 mmol/l CaCl₂ 50 mmol/l Mannit, 1 mmol/l NaDCh und 4 mmol/l Triton®X-100 versetzt. Die Substratkonzentration beträgt 17 mmol/l.

Die Emulsion wird 1 Stunde bei Raumtemperatur auf dem Magnetrührer gerührt. Die Extinktion der Emulsion wird bei 365 nm gegen Luft gemessen. Sie beträgt 1,851.

### Testansatz:

a) Inkubationsansatz: 250 »l der oben genannten Emulsion werden mit 50 »l Probe inkubiert. Nach 5 min bzw. 20 min werden jeweils 50 »l entnommen und in 50 »l Stopplösung pipettiert. Die Inkubation erfolgt bei 37°C.
b) Bestimmungsansatz: Wellenlänge: 546 nm gegen Luft
   Temperatur: 37°C
   Zu 1000 »l Reagenzlösung A (ATP, Acyl-CoA-Synthetase, Peroxidase, Ascorbatoxidase, 4-Aminoantipyrin) werden 100 »l Probe gegeben. Nach 10 min werden 50 »l der Reagenzlösung B (N-Ethylmaleinimid, Acyl-CoA-Oxidase) dazugegeben. Nach weiteren 10 min wird die Extinktion gemessen. Aus der Differenz ergibt sich die PLA-Aktivität.

Mit dem Substrat wird eine sehr gute Wiederfindung der PLA-Aktivität (130 %) im PLA-Humanserum im Vergleich zu Sojabohnenlecithin als Substrat erreicht. Die Wiederfindung von Schweinepancreas PLA beträgt 52,9 %.

### Beispiel 2.2.1

### Bestimmung in Gegenwart von Triethanolamin-Puffer

Die, wie im Beispiel 2.1. beschrieben, hergestellte Emulsion wird lyophilisiert und gelöst in 5,0 ml eines Triethanolamin-Puffers (100 mmol/l, pH 7,8), der zusätzlich 2,16 mg ACS, 4,34 mg CoA und 20,82 mg ATP enthält (in 5 ml).

Zu 500 »l dieser Lösung werden 100 »l Probelösung gegeben und 5 Minuten bei 37°C inkubiert. Anschließend werden 200 »l dieser Lösung zu 1 ml Reagenzlösung A (POD, 4-Aminoantipyrin), 50 »l Lösung B (N-Ethylmaleinimid, Acyl-CoA-Oxidase) und 500 »l einer 0,5 %igen Triton®X100 Lösung gegeben. Die Extinktion wird nach 10 Minuten bei 365 nm gegen Luft gemessen. Nach 20 Minuten wird die Extinktion erneut gemessen und aus der Differenz der Extinktionen die PLA-Aktivität ermittelt.

### Beispiel 2.2:

### PLA-Substrat:

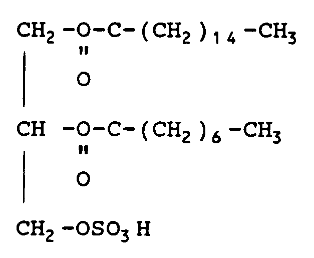

### Herstellung der Emulsion:

a) wie in Beispiel 1 angegeben. Die Extinktion der Emulsion beträgt 2,082 bei 365 nm gegen Luft.
b) Das oben genannte Substrat (Endkonzentration: 12 mmol/l) wird mit 1,35 ml HEPES-Puffer (100 mmol/l, pH = 7,9) versetzt, anschließend werden 0,075 ml 120 mmol/l Taurodesoxycholatlösung dazugegeben. Unter leichtem Erwärmen wird auf dem Magnetrührer 30 min gerührt. Zu dieser Emulsion werden 0,075 ml einer Lösung von 200 mmol/l CaCl₂·2 H₂O, 200 mmol/l Triton®X-100 und 400 mmol/ 1 MgCl₂·6 H₂O gegeben.
   Es wird eine klare Emulsion mit einer Extinktion von 0,222 bei 365 nm gegen Luft erhalten.

Testansatz: wie in Beispiel 1 angegeben.
Das Substrat ist zum Nachweis von Schweinepancreas-PLA und Serum-PLA geeignet. Die Schweinepancreas-PLA wird niedriger wiedergefunden als die Humanserum-PLA.

### Beispiel 2.3:

### PLA-Substrat:

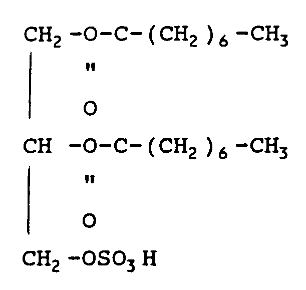

### Herstellung der Emulsion:

a) wie in Beispiel 1 angegeben. Die Extinktion der Emulsion beträgt 0,605.
b) wie in Beispiel 2 unter Punkt b) beschrieben.
   Die Substratkonzentration beträgt in beiden Fällen 12 mmol/l.

### Testansatz:

Wie in Beispiel 1 angegeben.

Das Substrat ist zum Nachweis von Schweinepancreas-PLA und humaner Serum-PLA geeignet, wobei Schweinepancreas-PLA niedriger wiedergefunden wird als Serum-PLA.

### Beispiel 2.4:

### PLA-Substrat:

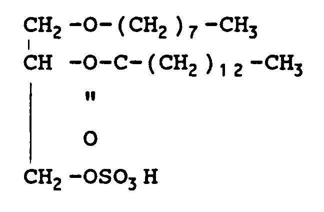

### Herstellung der Emulsion:

Wie in Beispiel 1 angegeben.

### Testansatz:

Wie in Beispiel 1 angegeben.

Das Substrat ist als PLA-Substrat geeignet.

### Beispiel 3

### Lecithine

Es wurden folgende Lecithine bezüglich ihrer Eignung als PLA-Substrat untersucht:
- Lecithin aus Sojabohnen, Roth 9812
- Lecithin aus Eiern, Merck 5331
- Lecithin ex cerebro, Roth 5957
- Lecithin aus Eidotter, Serva 27608
- Lipoid S 20

### Herstellung der Emulsion:

35 mg Lecithin werden mit 4,5 ml HEPES-Puffer (100 mmol/l, pH = 7,9) und 0,250 ml 120 mmol/l Taurodesoxycholatlösung versetzt und unter leichtem Erwärmen gerührt. Nachdem das Lecithin emulgiert ist (nach 80 - 110 min), werden 0,250 ml einer Lösung von 200 mmol/l CaCl₂·2 H₂O, 200 mmol/l Triton®X-100 und 400 mmol/l MgCl₂·6 H₂O hinzugefügt.

### Testansatz:

Die Bestimmungen werden bei 37°C durchgeführt. Gemessen wird bei einer Wellenlänge von 365 nm gegen Luft.
Zu jeweils 750 »l der oben genannten Emulsionen werden 25 »l einer CoA-Lösung (90 mmol/l), 75 »l einer Coenzymlösung (ATP: 145 mmol/l, PEP: 55 mmol/l, NADH: 9,4 mmol/l) sowie 50 »l einer Enzymlösung (Myokinase: 165 kU/l, LDH: 377 kU/l, PK: 141 kU/l, ACS: 5,6 kU/l) gegeben. Als Probe werden 50 »l eines PLA-haltigen Humanserums eingesetzt.

Als geeignet erwiesen sich zwei der oben genannten Lecithine:
Lecithin aus Sojabohnen (Roth 9812) und Lipoid S 20. Mit den anderen Lecithinen konnte mit dem oben genannten Testansatz keine PLA-Aktivität im Serum nachgewiesen werden.

## Patentansprüche

1. Verfahren zur Bestimmung von Phospholipase A in Körperflüssigkeiten, insbesondere Serum,
**dadurch gekennzeichnet,**
daß man die Körperflüssigkeit mit einer Mischung, enthaltend ein Fettsäurereste aufweisendes Substrat für die Phospholipase und mindestens zwei Emulgatoren verschiedener chemischer Klasse, nämlich mindestens einen neutralen Emulgator und ein Gallensäuresalz, inkubiert und die Menge der durch Substratspaltung gebildeten freien Fettsäuren nach an sich bekannten Methoden bestimmt.

2. Verfahren nach Anspruch 1,
**dadurch gekennzeichnet,**
daß man als Substrat natürliches Lecithin aus Sojabohnen oder Lipoid S20 einsetzt.

3. Verfahren nach Anspruch 1,
**dadurch gekennzeichnet,**
daß man ein künstliches Substrat einsetzt, das der allgemeinen Formel 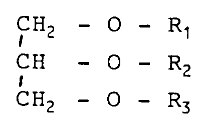 entspricht, wobei R₁ und R₂ einen Alkylrest mit bis zu 20 C-Atomen oder eine Carbonylgruppe mit bis zu 20 C-Atomen bedeuten, unter der Voraussetzung, daß mindestens einer der beiden Reste eine Carbonylgruppe ist, und R₃ -SO₃H bedeutet.

4. Verfahren nach einem der Ansprüche 1 bis 3,
**dadurch gekennzeichnet,**
daß man Emulgatoren aus der Klasse der Alkylglucoside, Polyether oder/und DMSO oder/und ein Desoxycholat oder/und Triton®X-100 verwendet.

5. Verfahren nach Anspruch 4,
**dadurch gekennzeichnet,**
daß man Taurodesoxycholat in Kombination mit einem zweiten Emulgator verwendet.

6. Verfahren nach Anspruch 5,
**dadurch gekennzeichnet,**
daß man eine Kombination von Taurodesoxycholat und Octylglucosid verwendet.

7. Verfahren nach Anspruch 5,
**dadurch gekennzeichnet,**
daß man eine Kombination von Taurodesoxycholat und Polyoxyethylenether verwendet.

8. Verfahren nach Anspruch 5,
**dadurch gekennzeichnet,**
daß man eine Kombination von Taurodesoxycholat und DMSO verwendet.

9. Verfahren nach Anspruch 5,
**dadurch gekennzeichnet,**
daß man eine Kombination von Taurodesoxycholat und Triton®X-100 verwendet.

10. Verfahren nach einem der vorhergehenden Ansprüche,
**dadurch gekennzeichnet,**
daß man die Bestimmung der gebildeten Fettsäuren durch Umsetzung der Fettsäuren in Gegenwart von Coenzym A und ATP mit Acyl-CoA-Synthetase zu Acyl-CoA, AMP und Pyrophosphat und nachfolgende Bestimmung einer der gebildeten Komponenten durchführt.

## Claims

1. Process for the determination of phospholipase A in body fluids, especially serum, characterised in that one incubates the body fluid with a mixture containing a substrate having fatty acid residues for the phospholipase and at least two emulsifiers of different chemical classes, namely, at least one neutral emulsifier and a bile acid salt, and determines according to per se known methods the amount of the free fatty acids formed by substrate cleavage.

2. Process according to claim 1, characterised in that one uses natural lecithin from soya beans or Lipoid S 20 as substrate.

3. Process according to claim 1, characterised in that one uses a synthetic substrate which corresponds to the general formula 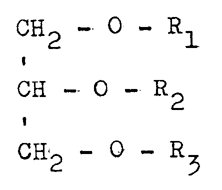 whereby R₁ and R₂ signifies an alkyl radical with up to 20 C-atoms or a carbonyl group with up to 20 C-atoms, with the proviso that at least one of the two radicals is a carbonyl group, and R₃ signifies -SO₃H.

4. Process according to one of claims 1 to 3, characterised in that one uses emulsifiers from the class of the alkyl gucosides, polyethers and/or DMSO and/or a desoxycholate and/or Triton®X-100.

5. Process according to claim 4, characterised in that one uses taurodesoxycholate in combination with a second emulsifier.

6. Process according to claim 5, characterised in that one uses a combination of taurodesoxycholate and octyl glucoside.

7. Process according to claim 5, characterised in that one uses a combination of taurodesoxycholate and polyoxyethylene ether.

8. Process according to claim 5, characterised in that one uses a combination of taurodesoxycholate and DMSO.

9. Process according to claim 5, characterised in that one uses a combination of taurodesoxycholate and Triton®X-100.

10. Process according to one of the preceding claims, characterised in that one carries out the determination of the fatty acids formed by reaction of the fatty acids in the presence of coenzyme A and ATP with acyl-CoA synthetase to give acyl-CoA, AMP and pyrophosphate and subsequent determination of one of the components formed.

## Revendications

1. Procédé pour la détermination de la phospholipase A dans les fluides corporels, en particulier dans le sérum, caractérisé en ce que l'on procède à l'incubation du fluide corporel avec un mélange contenant un substrat présentant un radical d'acide gras pour la phospholipase et au moins deux émulsifiants de différentes catégories chimiques, en l'occurrence au moins un émulsifiant neutre et un sel de l'acide bilaire, et en ce que l'on détermine la quantité des acides gras libres formés par division du substrat selon des procédés connus per se.

2. Procédé selon la revendication 1, caractérisé en ce que comme substrat on met en oeuvre de la lécithine naturelle à partir de soja ou de lipoide S20.

3. Procédé selon la revendication 1, caractérisé en ce que l'on met en oeuvre un substrat artificiel qui correspond à la formule générale 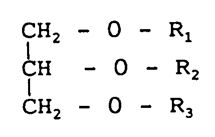 dans laquelle R₁ et R₂ signifient un radical alkyle avec jusqu'à 20 atomes C ou un groupe carbonyle avec jusqu'à 20 atomes C à condition qu'au moins l'un des deux radicaux signifie un groupe carbonyle et R₃ signifie SO₃H.

4. Procédé selon l'une des revendications 1 à 3, caractérisé en ce que l'on utilise des émulsifiants de la catégorie des alkylglucosides, des polyéthers ou/et DMSO ou/et un désoxycholate et/ou Triton®X-100.

5. Procédé selon la revendication 4, caractérisé en ce que l'on utilise un taurodésoxycholate en combinaison avec un deuxième émulsifiant.

6. Procédé selon la revendication 5, caractérisé en ce que l'on utilise une combinaison de taurodésoxycholate et d'octylglucoside.

7. Procédé selon la revendication 5, caractérisé en ce que l'on utilise un taurodésoxycholate en combinaison avec un polyoxyéthylène-éther.

8. Procédé selon la revendication 5, caractérisé en ce que l'on utilise un taurodésoxycholate en combinaison avec un DMSO.

9. Procédé selon la revendication 5, caractérisé en ce que l'on utilise un taurodésoxycholate en combinaison avec un Triton®X-100.

10. Procédé selon l'une des revendications précédentes, caractérisé en ce que l'on procède à la détermination des acides gras formés par transformation des acides gras en présence de coenzyme A et ATP avec l'acyl-CoA-synthétase en acyl-CoA, AMP et pyrophosphate et détermination subséquente de l'un des composants formés.
